(19) Europäisches Patentamt
European Patent Office
Office européen des brevets

(11) Publication number: 0 145 019 B1

(12) **EUROPEAN PATENT SPECIFICATION**

(45) Date of publication of patent specification: 07.11.90

(51) Int. Cl.$^5$: C 07 D 401/12, C 07 D 403/12, C 07 D 237/14, A 61 K 31/50, A 61 K 31/505

(21) Application number: 84115292.9

(22) Date of filing: 12.12.84

(54) **Pyridazinone derivatives and salts thereof.**

(30) Priority: 14.12.83 JP 235480/83
16.03.84 JP 50693/84

(43) Date of publication of application: 19.06.85 Bulletin 85/25

(45) Publication of the grant of the patent: 07.11.90 Bulletin 90/45

(84) Designated Contracting States: AT BE CH DE FR GB IT LI NL SE

(56) References cited: EP-A-0 084 250

(73) Proprietor: MITSUBISHI KASEI CORPORATION
5-2, Marunouchi 2-chome Chiyoda-ku
Tokyo 100 (JP)

(72) Inventor: Okushima, Hiromi
2747-3 Ohzenji, Asao-ku
Kawasaki-shi Kanagawa-ken (JP)
Inventor: Narimatsu, Akihiro
3-3 Tsutsujigaoka, Midori-ku
Yokohama-shi Kanagawa-ken (JP)
Inventor: Kobayashi, Makio
2-1-18 Tsurukawa, Machida-shi
Tokyo (JP)
Inventor: Shimooda, Isao
4095-7 Yatabe Hasaki-machi
Kashima-gun Ibaraki-ken (JP)
Inventor: Kitada, Yoshimi
6-20 Satsukigaoka, Midori-ku
Yokohama-shi Kanagawa-ken (JP)
Inventor: Furuya, Rikizo
2-12-1 Naruse
Machida-shi Tokyo (JP)

(74) Representative: TER MEER - MÜLLER - STEINMEISTER & PARTNER
Mauerkircherstrasse 45
D-8000 München 80 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 145 019 B1

**Description**

Background of the Invention

This invention relates to a noval pyridazinone derivative or a salt thereof which is useful for a cardiac stimulant.

Pyridazinone derivatives of the formula

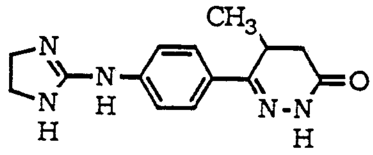 and 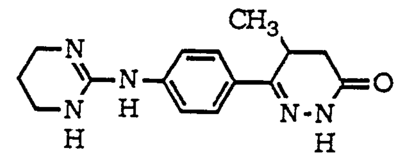

have been known as platelet aggregation inhibiting agents and blood pressure depressing compounds as disclosed in EP—A—0 084 250. However, they have never been employed as cardiac stimulants.

Various cardiac stimulants, which effect a direct enhancement of the cardiac contraction, have been available in the treatment of cardiac insufficiency. However, these cardiac stimulants may have some defects such as follows: (1) their safety margin is extremely small; (2) they may cause arrhythmia; (3) their cardiac stimulating activity is transient; and (4) they may not be suitable for oral administration.

A primary object of the present invention is to provide a novel cardiac compound having a high and sustained activity.

Summary of the Invention

In the course of the present inventors' study onto compounds which act as cardiac stimulants with a high and sustained activity, the present invention has been achieved. The present invention pertains to pyridazinone derivatives having the general formula (I):

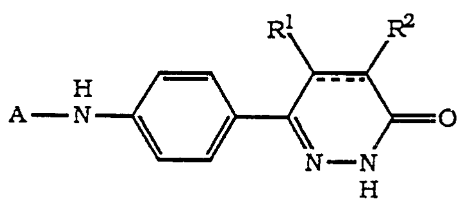 (I)

wherein A represents 5- or 6-membered aromatic heterocyclic ring having 1—3 nitrogen atoms, the ring being optionally substituted by at least a member selected from the group consisting of $C_{1-5}$ alkyl, cyano, hydroxyl, $C_{1-5}$ alkoxyl, amino, $C_{1-5}$ alkylamino, $C_{2-6}$ dialkyamino, $C_{2-5}$ acylamino, carboxyl, $C_{2-5}$, alkoxy-carbonyl and carbamoyl, $R^1$ and $R^2$ independently represent a hydrogen atom or $C_{1-5}$ alkyl or $R^1$ and $R^2$ may form together $C_{1-5}$ alkylene, and the dotted line represents either a single bond or a double bond, and a salt thereof with a pharmaceutically acceptable organic or inorganic acid.

Detailed Description of the Invention

The group A in the general formula (I) includes, for instance, pyridyl, pyridazinyl, pyrimidinyl, pyrazinyl, sym-trazinyl, asym-triazinyl, pyrrolyl, imidazolyl, and pyrazolyl, which may also be substituted by at least one substituent. By way of illustrating the substituent, mention may be made of normal or branched $C_{1-5}$ alkyl such as methyl, ethyl, propyl, butyl and pentyl; cyano; hydroxyl; normal or branched $C_{1-5}$ alkoxyl such as methoxy, ethoxy, propoxy and butoxy; amino; normal or branched $C_{1-5}$ alkylamino such as methylamino, ethylamino, propylamino and butylamino; normal or branched $C_{2-6}$ dialkylamino such as dimethylamino and diethylamino; normal or branched $C_{2-5}$ acylamino such as acetylamino, propionylamino and butrylamino; carboxyl; normal or branched $C_{2-5}$ alkoxycarbonyl such as methoxy-carbonyl, ethoxycarbonyl, propoxycarbonyl and butoxycarbonyl; and carbamoyl.

Preferably, A represents an imidazolyl substituted by a methyl and/or a hydroxyl, or a pyridyl substituted by at least a member selected from the group consisting of methyl, cyano, hydroxyl, methoxy, amino, dimethylamino and carbamoyl.

In a preferred pyridazinone derivative of this invention both $R^1$ and $R^2$ represent a hydrogen atom. In another preferred pyridazinone derivative both $R^1$ and $R^2$ are methyl.

Examples of pyridazinone derivatives having the general formula (I) are the following compounds:

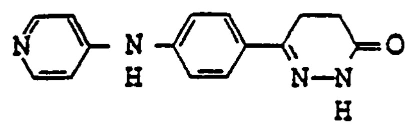

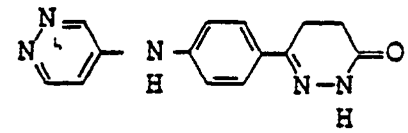

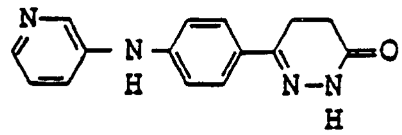

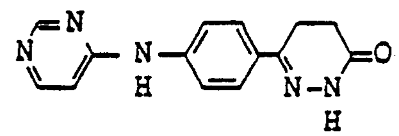

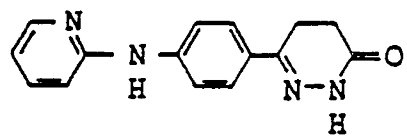

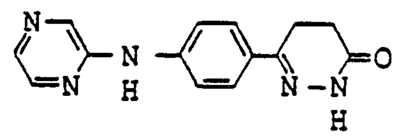

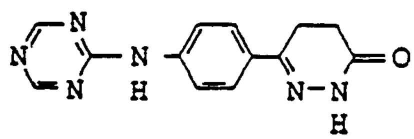

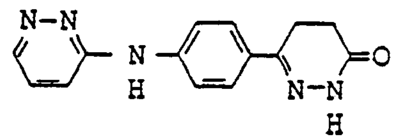

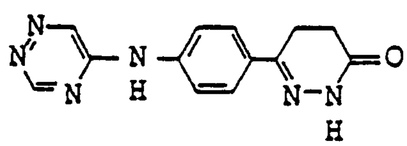

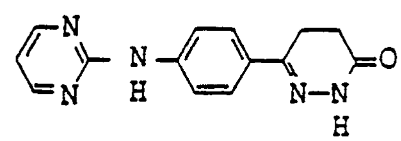

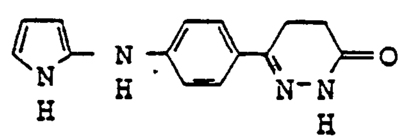

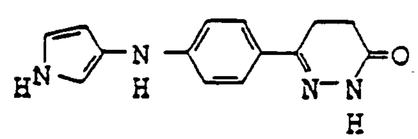

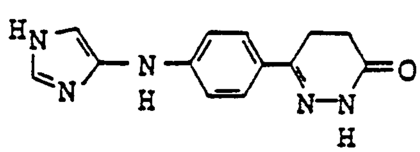

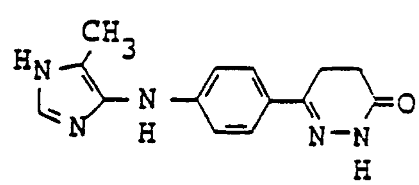

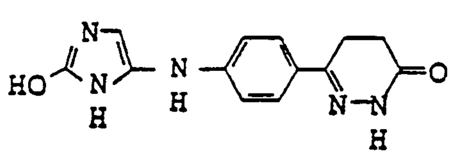

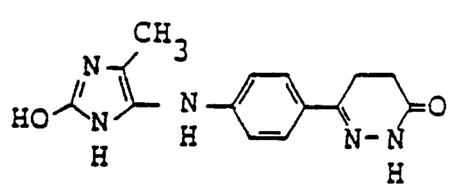

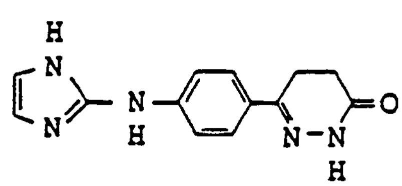

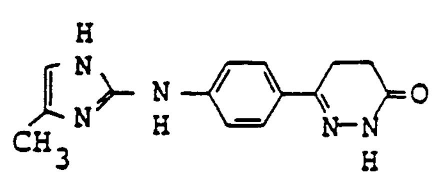

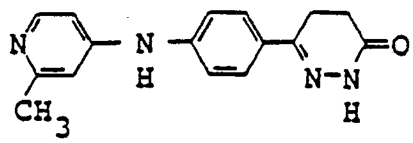
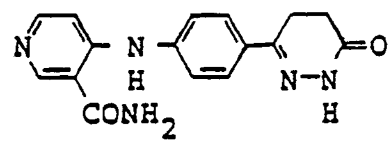

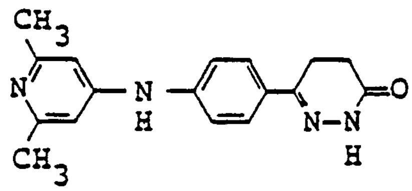
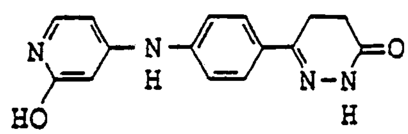

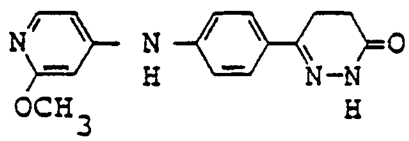
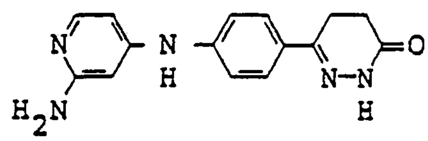

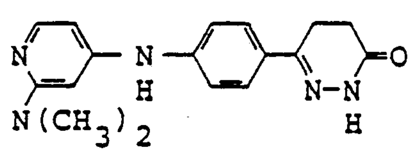
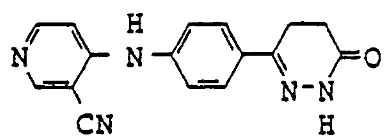

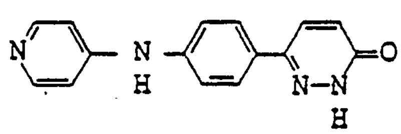
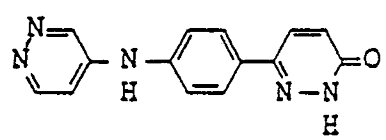

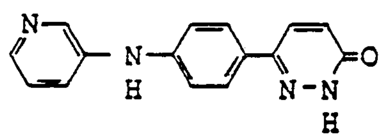
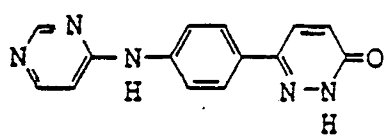

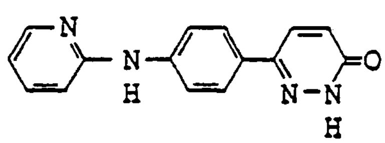
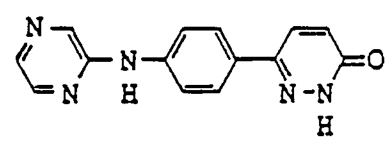

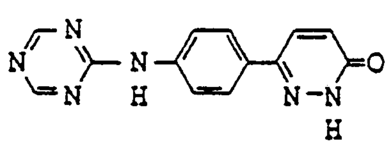
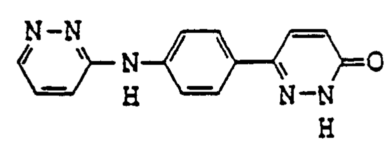

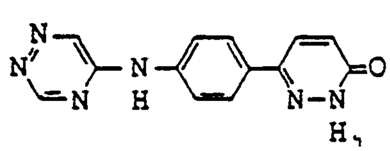
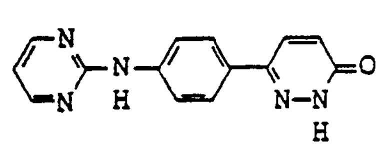

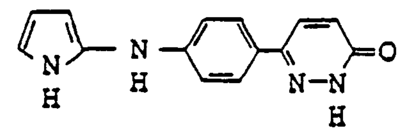
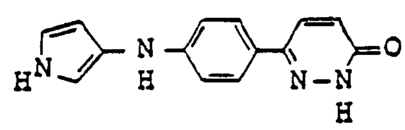

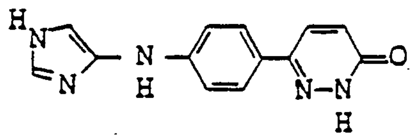
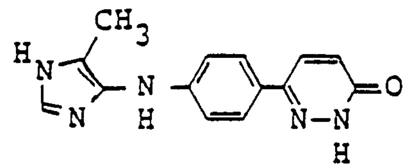

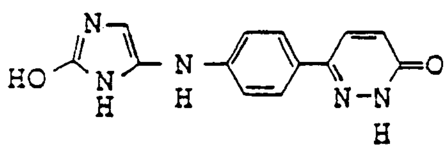
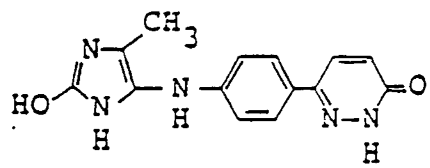

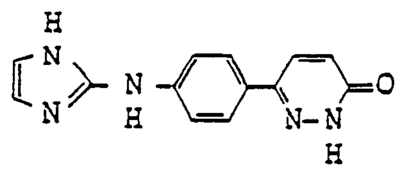
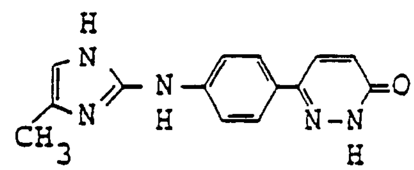

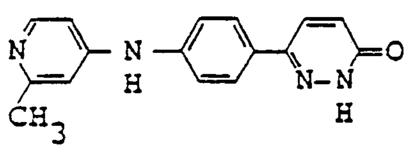
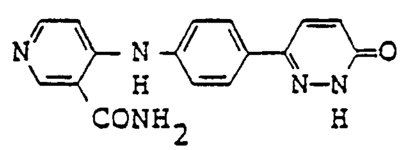

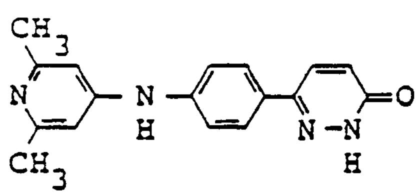
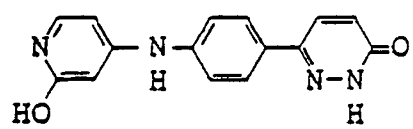

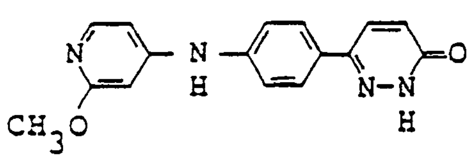
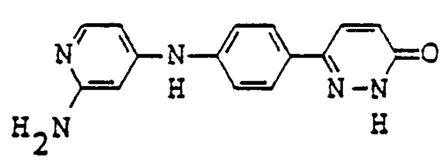

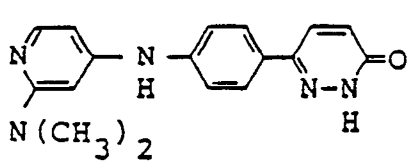
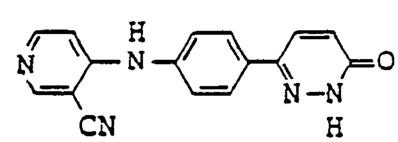

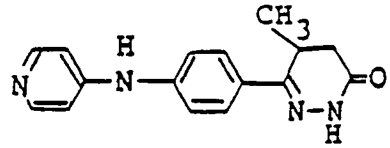

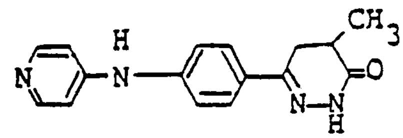

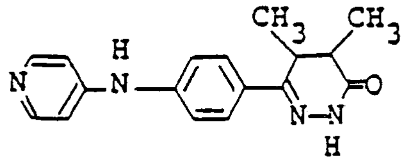

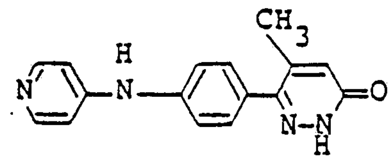

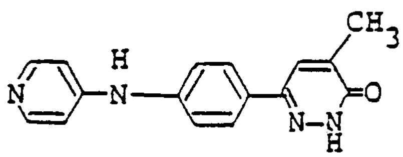

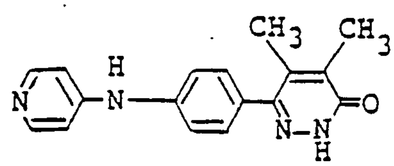

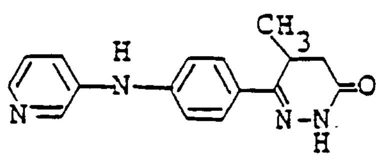

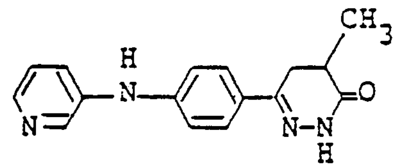

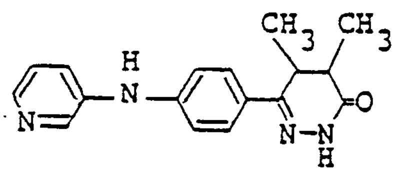

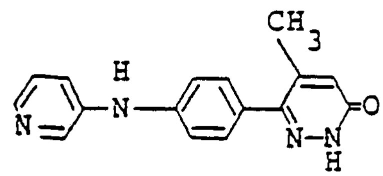

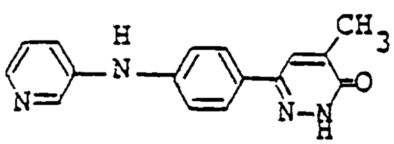

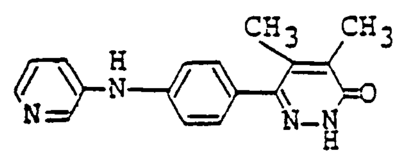

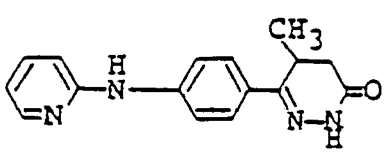

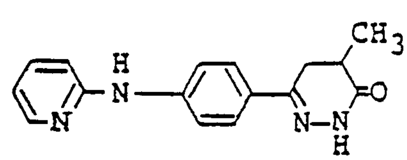

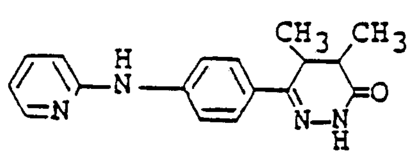

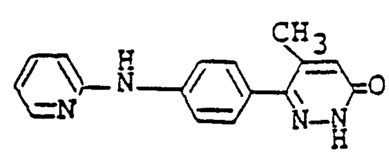

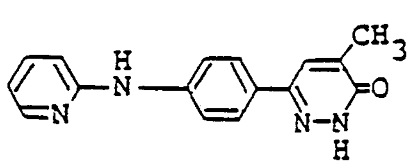

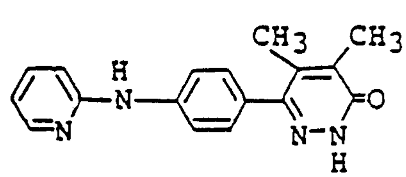

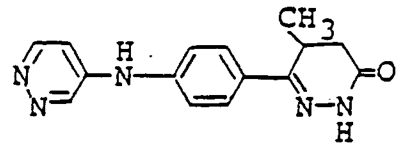

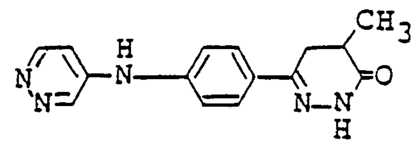

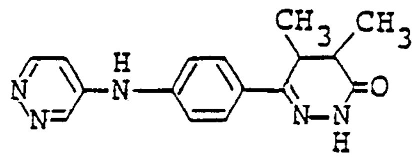

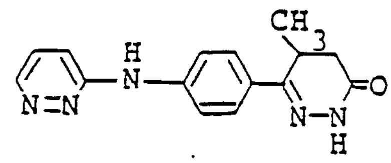

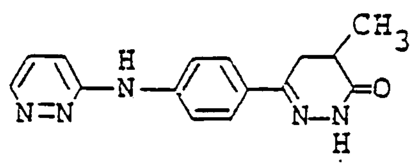

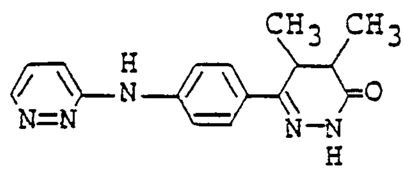

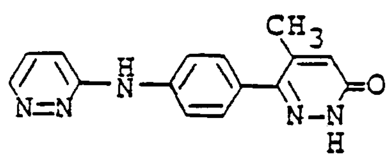

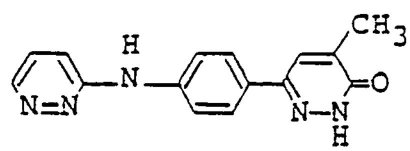

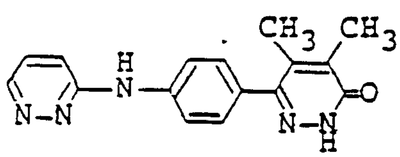

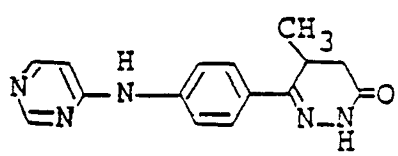

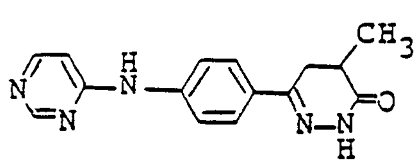

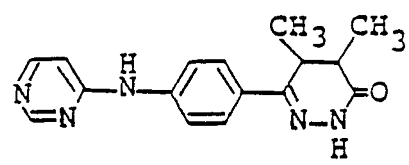

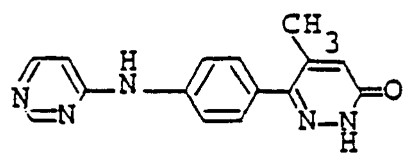

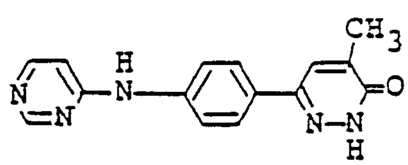

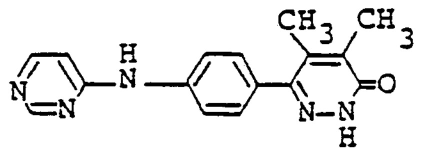

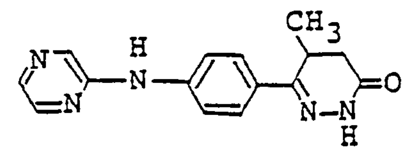

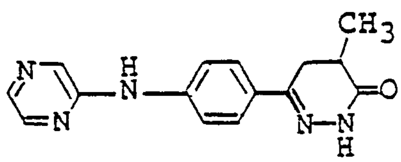

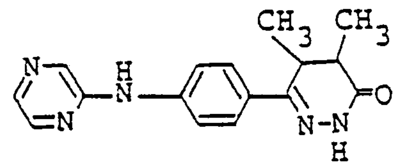

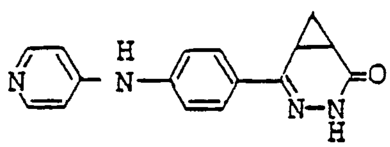

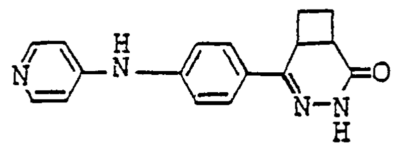

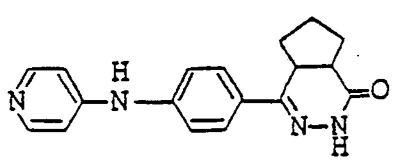

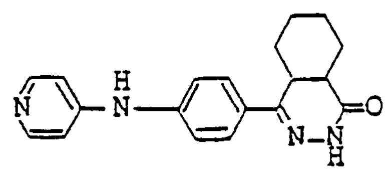

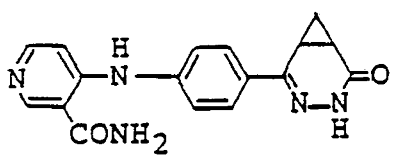

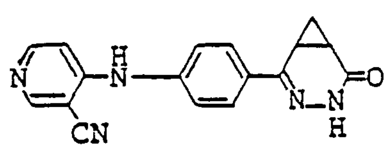

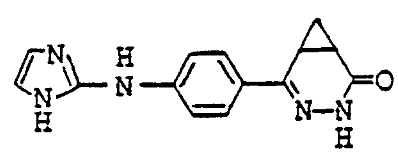

Pharmaceutically acceptable salts of pyridazinone derivatives having the general formula (I) are also included in the scope of the present invention. By illustrating those salts, mention may be made of salts of inorganic acid such as hydrochloric acid and phosphoric acid and of organic acid such as lactic acid and acetic acid. All these compounds are useful as cardiac stimulants.

Preparation of the compound according to the present invention will be described below.

A pyridazinone derivative according to the present invention, for example, may be prepared as follows:

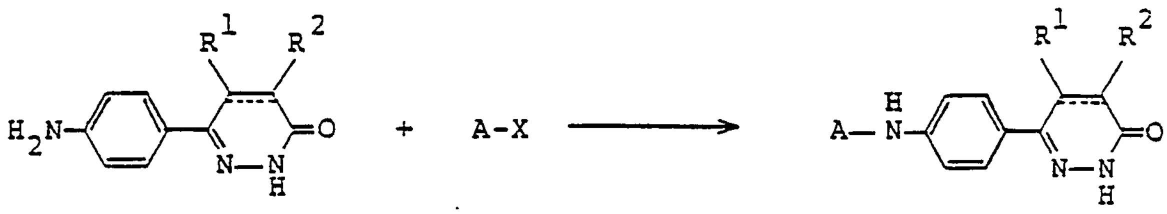

(II) (III) (I)

wherein A, $R^1$, $R^2$ and the dotted line are as defined hereinabove, and X represents a halogen atom.

That is, a desired pyridazinone derivative (I) may be synthesized by heating a mixture of a compound (II) and a compound (III) in a polar solvent such as dimethylformamide, dimethylacetamide and phenol at 50—200°C for about 0.5—10 hours. A copper compound may be used as a catalyst.

A compound (II) is a known compound described in Journal of Medicinal Chemistry, *17,* 273—280 (1974).

The compounds according to the present invention, when used as a cardiac stimulant, may be administered suitably by an oral and parenteral route. By way of illustrating available dosage unit form, mention may be made of powder, granule, tablet, sugar-coated tablet, pill, capsule and solution in case of oral administration, and suppository, suspension, solution, emulsion, ampoule and injectable solution in case of parenteral administration. A combination of these dosage forms may of course be also available. These dosage forms of the compound according to the present invention may be formulated pharmaceutically by the usual way in the art. Dosage can be determined by a physician according to age, sex, body weight, sensitivity to the drug, degree of sumptom and physical condition of a patient, administration route, duration and interval of administration, properties, formulation and type of pharmaceutical preparation, kind of active ingredient and so on. Dosage range, for example, is generally 0.01—30 mg/kg/day, and preferably approximately 0.05—10 mg/kg/day by oral administration.

The invention will be more clearly understood with reference to the following examples.

Example I

Preparation of 6-[4-(4′-pyridyl)aminophenyl]-4,5-dihydro-3(2H)-pyridazinone

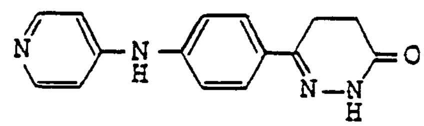

3.89 g of 4-bromopyridine hydrochloride and 3.78 g of 6-(4-aminophenyl)-4,5-dihydro-3(2H)-pyridazinone were dissolved in 50 ml of N,N-dimethylformamide, and then reacted for 2 hours at 105°C under the nitrogen gas flow. The reaction mixture was poured into 800 ml of water containing 2.12 g of sodium carbonate, and then the deposited crystal was filtered out, washed with water and dried under reduced pressure to recover 4.28 g of 6-[4-(4′-pyridyl)aminophenyl]-4,5-dihydro-3(2H)-pyridazinone in the yield of 70.7%. The crystal thus obtained was dissolved in methyl alcohol with heating, mixed with HCl-ethyl alcohol and then with ether to be converted into hydrochloride salt.

IR (KBr): 1642 $cm^{-1}$

MS: $M^{\oplus}$ 266

Example II

Preparation of 6-[4-(2′-pyrimidinyl)aminophenyl]-4,5-dihydro-3(2H)-pyridazinone

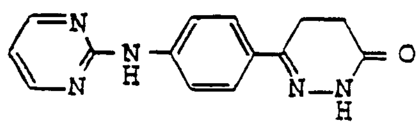

A mixture of 2 g of 6-(4-aminophenyl)-4,5-dihydro-3(2H)-pyridazinone and 1.22 g of 2-chloropyrimidine was heated under reflux in 10 ml of dimethylformamide for 4 hours and cooled to deposit crystal. The deposited crystal was filtered out, washed with dimethylformamide and tetrahydrofuran, and dried to recover 1.20 g of 6-[4-(2′-pyrimidinyl)aminophenyl)-4,5-dihydro-3(2H)-pyridazinone in the yield of 42.5%.

IR (KBr): 1670 $cm^{-1}$

Example III

Preparation of 6-[4-(4′-pyridyl)aminophenyl]-4-methyl-4,5-dihydro-3(2H)-pyridazinone

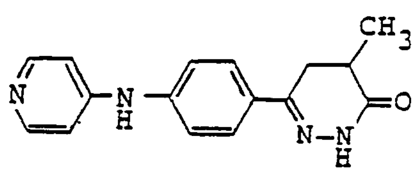

1.02 g of 6-(4-aminophenyl)-4-methyl-4,5-dihydro-3(2H)-pyridazinone was dissolved in 5 ml of N-methylpyrrolidone, to which 0.35 ml of triethylamine was added and then heated to 90°C. 0.75 g of 4-chloropyridine hydrochloride was added and reacted at 90°C for 2 hrs. After the reaction mixture was

cooled on ice, 50 ml of acetone was added. The deposited crystal was filtered out and dissolved in 50 ml of water. The aqueous solution was adjusted to approximate pH 9 with 1 N NaOH. The deposited crystal was collected by decantation and washed with acetone and n-hexane. Silica gel chromatography of the crystal thus obtained which was dissolved in 10 ml of N,N-dimethylformamide was performed with chloroform/methanol. The fraction of the product was concentrated and dried, and the residue was dissolved in ethanol and mixed with 1N HCl/ethanol to convert into hydrochloride salt. Benzene, hexane, and ethyl acetate were added to the solution to deposit hydrochloride salt which was filtered out and dried to obtain 0.98 g of 6-[4-(4'-pyridyl)aminophenyl-4-methyl-4,5-dihydro-3(2H)pyridazinone in the yield of 61.6%.

m.p.=241—244°C

Example IV

Preparation of 6-[4-(4'-pyridyl)aminophenyl]-5-methyl-4,5-dihydro-3(2H)-pyridazinone

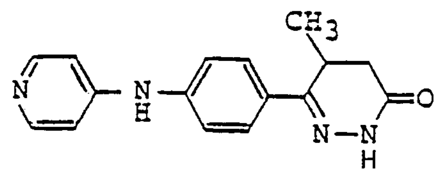

0.81 g of 6-(4-aminophenyl)-5-methyl-4,5-dihydro-3(2H)-pyridazinone was dissolved in 4 ml of N-methylpyrrolidone, mixed with 0.28 ml of triethylamine and then heated to 90°C. 0.60 g of 4-chloropyridine hydrochloride was added and reacted at 90°C for 2 hrs. The reaction mixture was cooled on ice bath and mixed with 50 ml of acetone to deposit crystal. The crystal was dissolved in water and alkalinized with 1N NaOH to deposit the crystal which was then performed silica gel chromatography with chloroform/methanol. The residue which was obtained by the concentration to dryness of the fractions was dissolved in ethyl alcohol and mixed with 1N HCl/ethanol to convert into hydrochloride salt. The ethanol solution was mixed with ether to deposit the crystal which was filtered out and dried to obtain 0.863 g of 6-[4-(4'-pyridyl)aminophenyl]-5-methyl-4,5-dihydro-3(2H)pyridazinone in the yield of 68.3%.

m.p.=249—252°C.

Example V

Preparation of 6-[4-(4'-pyridyl)aminophenyl]-3(2H)-pyridazinone

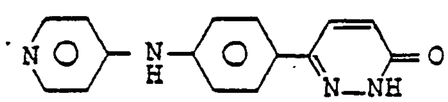

A mixture of 2.33 g of 4-bromopyridine hydrochloride and 1.87 g of 6-(4-aminophenyl)-3(2H)-pyridazinone was reacted in 30 ml of N,N-dimethylformamide under the nitrogen gas flow at 110°C for 4 hours.

After cooling the reaction mixture, 75 ml of water was added. The reaction mixture was neutralized with 2N sodium hydroxide followed by the filtration of deposited crystal.

The crude crytal thus obtained was recrystallized from a mixed solvent of ethanol-water and thoroughly purified with a silica gel chromatography to remove a trace amount of impurity contained in the crystal.

Fractions containing a desired substance were gathered, and the solvent was evaporated. Addition of HCl-ethanol and of ether of the condensate dissolved in a small amount of methanol yielded 0.23 g of 6-[4-(4'-pyridyl)aminophenyl]-3(2H)-pyridazinone hydrochloride.

IR (KBr): 1650 $cm^{-1}$

Example VI

Pharmacological and toxicological studies of pyridazinone derivatives according to the present invention were carried out by the following methods to show their utility as a cardiac stimulant.

1. Effect on contraction of an isolated and cross-circulated papillary muscle preparation of the dog

An isolated and cross-circulated papillary muscle preparation of the dog was prepared by Endo and Hashimoto's method (referred to American Journal of Physiology, *218*, 1459—1463, 1970). The effect of the compound was measured by closely intraarterially injecting the compound dissolved in a solvent to the papillary muscle for the purpose of recording its effect on contraction of papillary muscle. Rate of increase in contraction of papillary muscle is shown in Table 1.

2. Effect on contraction of an isolated left atrium of the guinea pig

A left atrium was isolated from a male guinea pig with 200—300 g of body weight soon after striking on the back of its head. The mitral orifice was fixed to the bottom of an organ bath filled with 30 ml of Krebs-Henseleit solution maintained at 35°C. A gas mixture comprising 95% of $O_2$ and 5% of $CO_2$ was passed through the Krebs-Henseleit solution in the organ bath. Isometric tension was measured by connecting a left auricle of heart and a transducer by a yarn. A resting tension of 0.5 g was given to the atrium, which was then electrically driven with square pulses with duration of 1 msec and voltage of 1.5 times as much as threshold in the rate of 2Hz via dipolar platinum electrodes.

After stabilizing the atrium for 30 minutes from its preparation, the compound dissolved in a solvent was added to the organ bath to measure its effect. The rate of increase in contraction of left atrium is shown in Table 1.

3. Effects on myocardial contraction in anesthetized dogs

Male and female mongrel dogs with body weight of 8—15 kg were used. A dog was anesthetized by intravenous injection of 30 mg/kg of sodium pentobarbital and practiced artificial respiration. The dog was thoractomized between fourth and fifth costa which was cut off. The pericardium was incised to expose heart. Blood flow through the aorta, which was measured with an electromagnetic blood flowmeter whose probe was attached to the ascending aorta, was used as an approximate index of cardiac output (CO). Left ventricular pressure (LVP) was measured with a Miller Catheter-tip pressure transducer and the first derivative of the LVP (dP/dt) was measured with a differentiater. Contraction of right ventricular muscle (Cont) was determined with a strain-gauge attached to the wall. Systemic blood pressure was measured from the left femoral artery. Heart rate was measured with electrocardiogram (lead II) and a cardiotachometer. The compound dissolved in a solvent was administered intravenously from a left femoral vein.

Maximum value of dP/dt (dP/dt max) and rate of increase in Cont and CO are shown in Table 1.

4. Acute Toxicity

Acute toxicity ($LD_{50}$) for intravenous and oral administration in male mice was determined by the method of Richfield and Wilcoxon (referred to Journal of Pharmacology and Experimental Therapeutics, *96,* 99—113, 1949). The result is shown in Table 1.

TABLE 1

| Compound | Contraction of pipillary muscle of dog | | Contraction of left atrium of guinea pig | | Anesthetized dog | | | | $LD_{50}$ (mg/kg) | |
|---|---|---|---|---|---|---|---|---|---|---|
| | dosage (μg i.a.) | increase (%) | dosage (g/ml) | increase (%) | dosage (μg/kg i.v.) | dP/dt max increase (%) | Cont increase (%) | CO increase (%) | i.v. | p.o. |
| 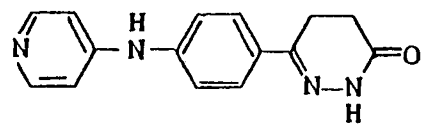 Example I | 10 | 13.2 | $10^{-5}$ | 115 | 10 | 45 | 23 | 13 | 97 | 341 |
| | 30 | 15.4 | $3\times10^{-5}$ | 138 | 30 | 93 | 57 | 28 | | |
| 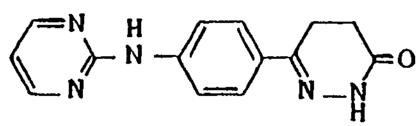 Example II | 30 | 5.7 | $10^{-5}$ | 42 | | | | | | |
| | 100 | 15.9 | $3\times10^{-5}$ | 66 | | | | | | |
| 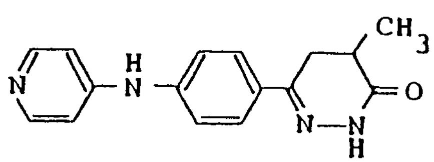 Example III | 10 | 22.6 | $10^{-5}$ | 85.9 | 30 | 29.1 | 21.6 | 8.6 | | |
| | 30 | 50.0 | $3\times10^{-5}$ | 109.3 | 100 | 64.7 | 64.0 | 26.7 | | |

TABLE 1

| Compound | Contraction of pipillary muscle of dog | | Contraction of left atrium of guinea pig | | Anesthetized dog | | | | $LD_{50}$ (mg/kg) | |
|---|---|---|---|---|---|---|---|---|---|---|
| | dosage (μg i.a.) | increase (%) | dosage (g/ml) | increase (%) | dosage (μg/kg i.v.) | dP/dt max increase (%) | Cont increase (%) | CO increase (%) | i.v. | p.o. |
| 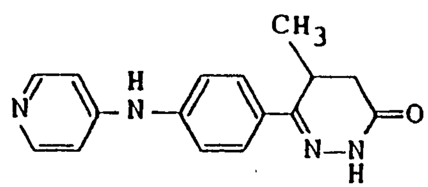 Example IV | 3 | 28.6 | $10^{-5}$ | 86.6 | 3 | 14.4 | 13.3 | 14.4 | | |
| | 10 | 38.7 | $3\times10^{-5}$ | 97.9 | 10 | 73.8 | 75.9 | 26.7 | | |
| 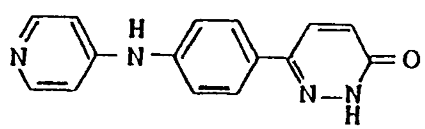 Example V | 30 | 12.4 | $10^{-5}$ | 163 | 30 | 64 | 31 | 13 | 103 | |
| | | | | | 100 | 108 | 39 | 11 | | |

**Claims**

1. A pyridazinone derivative having the general formula (I):

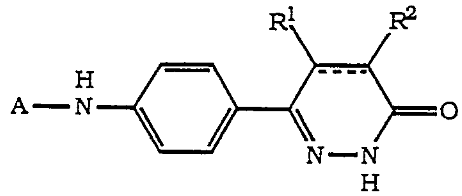

(I)

wherein A represents a 5- or 6-membered aromatic heterocyclic ring having 1—3 nitrogen atoms, the ring being optionally substituted by at least a member selected from the group consisting of $C_{1-5}$ alkyl, cyano, hydroxyl, $C_{1-5}$ alkoxyl, amino, $C_{1-5}$ alkylamino, $C_{2-6}$ dialkylamino, $C_{2-5}$ acylamino, carboxyl, $C_{2-5}$ alkoxycarbonyl and carbamoyl, $R^1$ and $R^2$ independently represent hydrogen or $C_{1-5}$ alkyl or $R^1$ or $R^2$ may form together $C_{1-5}$ alkylene, and the dotted line represents either a single bond or a double bond, and a salt thereof with a pharmaceutically acceptable organic or inorganic acid.

2. A pyridazinone drivative as set forth in claim 1, wheerin A represents a member selected from the group consisting of pyridyl, pyridazinyl, pyrimidinyl, pyrazinyl, sym-triazinyl, asym-triazinyl, pyrrolyl, imidazolyl and pyrazolyl.

3. A pyridazinone derivative as set forth in claim 1, wherein A is substituted by at least a member selected from the group consisting of $C_{1-5}$ alkyl, cyano, hydroxyl, $C_{1-5}$ alkoxyl, amino, $C_{1-5}$ alkylmaino, $C_{2-6}$ dialkylamino, $C_{2-5}$ acylamino, carboxyl, $C_{2-5}$ alkoxycarbonyl and carbamoyl.

4. A pyridazinone derivative as set forth in claim 1, wherein A represents an imidazolyl substituted by a methyl and/or a hydroxyl, or a pyridyl substitued by at least a member selected from the group consisting of methyl, cyano, hydroxyl, methoxy, amino, dimethylamino and carbamoyl.

5. A pyridazinone derivative as set forth in claim 1, wherein both $R^1$ and $R^2$ represent a hydrogen atom.

6. A pyridazinone derivative as set forth in claim 1, wherein $R^1$ or $R^2$ represents methyl.

7. 6-[4-(4'-pyridyl)aminophenyl]-4,5-dihydro-3(2H)-pyridazinone.

8. 6-[4-(2'-pyrimidinyl)aminophenyl]-4,5-dihydro-3(2H)-pyridazinone.

9. 6-[4-(4'-pyridyl)aminophenyl]-4-methyl-4,5-dihydro-3(2H)-pyridazinone.

10. 6-[4-(4'-pyridyl)aminophenyl]-5-methyl-4,5-dihydro-3(2H)-pyridazinone.

11. 6-[4-(4'-pyridyl)aminophenyl]-3(2H)-pyridazinone.

12. A process for preparing a pyridazinone derivative having the general formula (I):

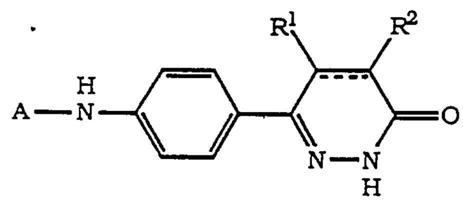

(I)

which comprises reacting a compound (II) with a compound (III):

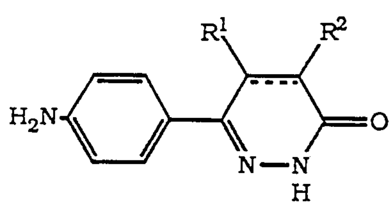

(II)

A–X (III)

wherein A represents 5- or 6-membered aromatic heterocyclic ring having 1—3 nitrogen atoms, the ring being optionally substituted by at least a member selected from the group consisting of $C_{1-5}$ alkyl, cyano, hydroxyl, $C_{1-5}$ alkoxyl, amino, $C_{1-5}$ alkylamino, $C_{2-6}$ dialkyamino, $C_{2-5}$ acylamino, carboxyl, $C_{2-5}$, alkoxycarbonyl and carbamoyl, $R^1$ and $R^2$ independently represent a hydrogen atom or $C_{1-5}$ alkyl or $R^1$ and $R^2$ may form together $C_{1-5}$ alkylene, the dotted line represents either a single bond or a double bond and X

represents a halogen atom, and optionally forming a salt with a pharmaceutically acceptable organic or inorganic acid.

13. A pharmaceutical composition useful for a cardiac stimulant containing the compound as set forth in any one of claims 1—11 together with a pharmaceutically acceptable carrier.

**Patentansprüche**

1. Pyradazinoderivat mit der allgemeinen Formel (I):

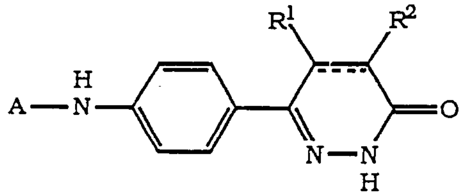

(I)

worin A einen 5- oder 6-gliedrigen aromatischen heterocyclischen Ring mit 1—3 Stickstoffatomen, wobei der Ring wahlweise mit mindestens einem Vertreter der $C_{1-5}$-Alkyl-, Cyano-, Hydroxyl-, $C_{1-5}$-Alkoxyl-, Amino-, $C_{1-5}$-Alkylamino-, $C_{2-6}$-Dialkylamino, $C_{2-5}$-Acylamino, Carboxyl-, $C_{2-5}$-Alkoxycarbonyl und Carbamoyl-gruppen umfassenden Gruppen substituiert ist, $R^1$ und $R^2$ voneinander unabhängig Wasserstoffatome oder $C_{1-5}$-Alkylgruppen bedeuten oder $R^1$ und $R^2$ zusammen $C_{1-5}$-Alkylen bilden können und die gestrichelte Linie entweder eine Einfachbingung oder eine Doppelbindung bedeutet und ein mit einer pharmazeutisch annehmbaren organischen oder anorganischen Säure gebildetes Salz davon.

2. Pyridazinonderivat nach Anspruch 1, worin A ein Vertreter der Gruppe Pyridyl-, Pyridazinyl-, Pyrimidinyl, Pyrazinyl, sym-Triazinyl-, asym-Triazinyl-, Pyrrolyl-, Imidazolyl- und Pyrazolylgruppen bedeutet.

3. Pyridazinonderivat nach Anspruch 1, worin Amit mindestens einem Vertreter der Gruppe $C_{1-5}$-Alkyl-, Cyano-, Hydroxyl-, $C_{1-5}$-Alkoxyl-, Amino-, $C_{1-5}$-Alkylamino-, $C_{2-6}$-Dialkylamino-, $C_{2-5}$-Acylamino-, Carboxyl-, $C_{2-5}$-Alkoxycarbonyl- und Carbamoylgruppen substituiert ist.

4. Pyridazinonderivat nach Anspruch 1, worin A eine Imidazolylgruppe bedeutet, welche mit einer Methyl- und/oder Hydroxylgruppe substituiert ist oder eine Pyridylgruppe, die mit mindestens einem Vertreter der Methyl-, Cyano-, Hydroxyl-, Methoxy-, Amino-, Dimethylamino- und Carbamoylgruppe n umfassenden Gruppensubstituiert ist.

5. Pyridazinonderivat nach Anspruch 1, worin sowohl $R^1$ als auch $R^2$ ein Wasserstoffatom bedeuten.

6. Pyridazinonderivat nach Anspruch 1, worin $R^1$ oder $R^2$ eine Methylgruppe bedeutet.

7. 6-[4-(4'-Pyridyl)-aminophenyl]-4,5-dihydro-3(2H)-pyridazinon.

8. 6-[4-(2'-Pyrimidinyl)-aminophenyl]-4,5-dihydro-3(2H)-pyridazinon.

9. 6-[4-(4'-Pyridyl)-aminophenyl]-4-methyl-4,5-dihydro-3(2H)-pyridazinon.

10. 6-[4-(4'-Pyridyl)-aminophenyl]-5-methyl-4,5-dihydro-3(2H)-pyridazinon.

11. 6-[4-(4'-Pyridyl)-aminophenyl]-3(2H)-pyridazinon.

12. Verfahren zur Herstellung eines Pyridazinonderivats mit der allgemeinen Formel (I):

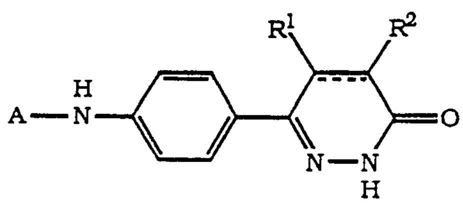

(I)

welches darin besteht, daß man eine Verbindung (II) mit einer Verbindung (III)

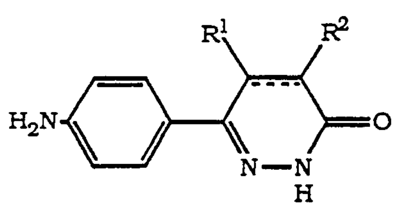

(II)

A–X (III)

worin A einen 5- oder 6-gliedrigen aromatischen heterocyclischen Ring mit 1—3 Stickstoffatomen, wobei

der Ring wahlweise mit mindestens einem Vertreter der $C_{1-5}$-Alkyl-, Cyano-, Hydroxyl-, $C_{1-5}$-Alkoxyl-, Amino-, $C_{1-5}$-Alkylamino-, $C_{2-6}$-Dialkylamino-, $C_{2-5}$-Acylamino-, Carboxyl-, $C_{2-5}$-Alkoxycarbonyl- und Carbamoylgruppe umfassenden Gruppen substituiert ist, $R^1$ und $R^2$ voneinander unabhängig Wasserstoffatome oder $C_{1-5}$-Alkylgruppen bedeuten oder $R^1$ und $R^2$ zusammen $C_{1-5}$-Alkylen bilden können, die gestrichelte Linie entweder eine Einfachbindung oder eine Doppelbindung bedeutet und X ein Halogenatom bedeutet, umsetzt und gegebenenfalls mit einer pharmazeutisch annehmbaren organischen oder anorganischen Säure ein Salz bildet.

13. Pharmazeutische Zusammensetzung für ein Herz-Stimulanz, das eine Verbindung nach einem der Ansprüche 1—11 zusammen mit einem pharmazeutisch annehmbaren Trägerstoff enthält.

**Revendications**

1. Dérivé de pyridazinone ayant la formule générale (I):

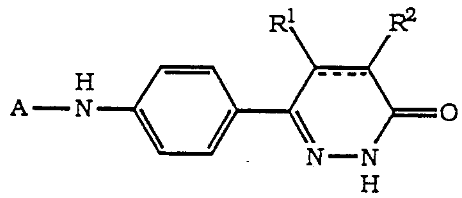

(I)

dans laquelle A représente un noyau aromatique hétérocyclique à 5 ou 6 chaînons ayant 1 à 3 atomes d'azote, le cycle étant éventuellement substitué par au moins un substituant choisi dans le groupe comprenant un groupe alkyle en $C_{1-5}$, cyano, hydroxy, alcoxy en $C_{1-5}$, amino, alkyl (en $C_{1-5}$)-amino, dialkyl (en $C_{2-6}$)-amino, acyl (en $C_{2-5}$)-amino, carboxy, alcoxy (en $C_{2-5}$)-carbonyle et carbamoyle; $R^1$ et $R^2$ représentent indépendamment un atome d'hydrogène ou un groupe alkyle en $C_{1-5}$ ou bien $R^1$ et $R^2$ peuvent former ensemble un radical alkylène en $C_{1-5}$ et la ligne en pointillés représente une liaison simple ou double, et un sel de celui-ci formé avec un acide organique ou inorganique acceptable du point de vue pharmaceutique.

2. Dérivé de pyridazinone suivant la revendication 1, caractérisé en ce que A est un élément choisi parmi les groupes pyridyle, pyridazinyle, pyrimidinyle, pyrazinyle, sym-triazinyle, asym-triazinyle, pyrrolyle, imidazolyle et pyrazolyle.

3. Dérivé de pyridazinone suivant la revendication 1, caractérisé en ce que A est substitué par au moins un élément choisi parmi un groupe alkyle en $C_{1-5}$, cyano, hydroxy, alcoxy en $C_{1-5}$; amino, alkyl (en $C_{1-5}$)-amino, dialkyl (en $C_{2-6}$)-amino, acyl (en $C_{2-5}$)-amino; carboxy, alcoxy (en $C_{2-5}$)-carbonyle et carbamoyle.

4. Dérivé de pyridazinone suivant la revendication 1, caractérisé en ce que A représente un groupe imidazolyle substitué par un groupe méthyle et/ou hydroxy, ou un groupe pyridyle substitué par au moins un élément choisi dans le groupe consistant en groupes méthyle, cyano, hydroxy, méthoxy, amino, diméthylamino, et carbamoyle.

5. Dérivé de pyridazinone suivant la revendication 1, caractérisé en ce que $R^1$ et $R^2$ représentent tous deux un atome d'hydrogène.

6. Dérivé de pyridazinone suivant la revendication 1, caractérisé en ce que $R^1$ et $R^2$ représentent tous deux un groupe méthyle.

7. 6-[4-(4'-pyridyl)aminophényl]-4,5-dihydro-3(2H)-pyridazinone.

8. 6-[4-(2'-pyrimidinyl)aminophényl]-4,5-dihydro-3(2H)-pyridazinone.

9. 6-[4-(4'-pyridyl)aminophényl]-4-méthyl-4,5-dihydro-3(2H)-pyridazinone.

10. 6-[4-(4'-pyridyl)aminophényl]-5-méthyl-4,5-dihydro-3(2H)-pyridazinone.

11. 6-[4-(4'-pyridyl)aminophényl]-3(2H)-pyridazinone.

12. Procédé de préparation d'un dérivé de pyridazinone ayant la formule générale (I):

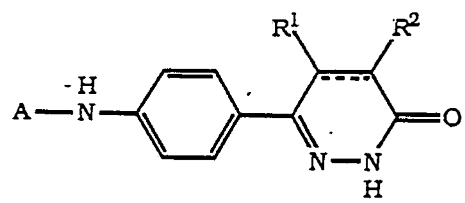

(I)

caractérisé en ce qu'il comprend la réaction d'un composé (II) avec un composé (III):

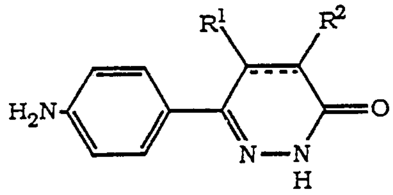

(II)

A–X (III)

dans lesquelles A représente un noyau aromatique hétérocyclique à 5 ou 6 chaînons ayant 1 à 3 atomes d'azote, le cycle étant éventuellement substitué par au moins un substituant choisi dans le groupe comprenant un groupe alkyle an $C_{1-5}$, cyano, hydroxy, alcoxy en $C_{1-5}$, amino, alkyl (en $C_{1-5}$), dialkyl (en $C_{2-6}$)-amino, acyl (en $C_{2-5}$)-amino, carboxy, alcoxy (en $C_{2-5}$)-carbonyle et carbamoyle; $R^1$ et $R^2$ représentent indépendamment un atome d'hydrogène ou un groupe alkyle en $C_{1-5}$ ou bien $R^1$ et $R^2$ peuvent former ensemble un radical alkylène en $C_{1-5}$ et la ligne en pointillés représente une liaison simple ou double et X représente un atome d'halogène, et la formation éventuelle d'un sel avec un acide organique ou inorganique acceptable du point de vue pharmaceutique.

13. Composition pharmaceutique utile pour un stimulant cardiaque, caractérisée en ce qu'elle contient le composé suivant l'une quelconque des revendications 1 à 11 avec un support acceptable du point de vue pharmaceutique.